# EUROPEAN PATENT APPLICATION

(11) **EP 4 461 249 A1**
(43) Date of publication of application: **13.11.2024**
(21) Application number: 23172535.9
(22) Date of filing: 10.05.2023
(51) Int. Cl.: A61B 34/10

(54) **TECHNIQUE FOR GENERATING AN IMAGE OF AN ANATOMICAL ELEMENT IN A POSE INDICATED BY PLANNING DATA**

(71) Applicant: Stryker European Operations Limited, Carrigtwohill, Co. Cork T45 HX08 (IE)
(72) Inventor: Collicott, Cristina, 67551 Worms (DE); Carvalho de Freitas Martins, Maria Manuel, 68420 Eguisheim (FR)
(74) Representative: Schott, Jakob Valentin

(57) **Abstract**

Disclosed is a technique for generating an image including a representation of an anatomical element of a patient's body, the method being performed by a computing system and comprising: obtaining medical image data of at least one anatomical element of a patient's body; obtaining planning data indicative of at least three planned positions relative to the at least one anatomical element, wherein one or more of the planned positions are associated with at least one planned medical implant; and generating, based on the medical image data and the planning data, an image comprising a representation of the at least one anatomical element in a pose defined by the at least three planned positions. A system, a computer program and a carrier medium are also disclosed.

## Description

The present disclosure generally relates to a method for generating an image including a representation of an anatomical element of a patient's body, a system, a computer program and a carrier medium.

### Background

Medical image data of a patient, for example X-ray image data, magnetic resonance (MR) image data or computer tomography (CT) image data, may be used for planning a surgical procedure. For instance, the placement of medical implants such as bone screws, bone plates, spinal cages, pedicle screws or the like relative to the patient's body may be planned by a surgeon based on pre-operative medical image data of the patient's body, before the surgical procedure is performed. During a later stage, a user may wish to be provided with an image that represents a portion of the patient's body and, optionally, the planned implant(s).

It may be desirable that the portion of the patient's body has a predefined pose in the image. For example, in the field of spinal surgery, surgeons are mainly interested in an image in which a vertebra of the patient's body is shown with the spinous process extending vertically upwards. In some solutions, an image including the vertebra may be provided and a user may change the rotation of the image to orient the vertebra in the predefined pose. Other solutions rely on complex segmentation algorithms to determine an orientation of a particular portion of the patient's body as represented in the medical image data.

### Summary

There is a need for a technique that solves one or more of the aforementioned or other problems.

According to a first aspect, a method for generating an image including a representation of an anatomical element of a patient's body is provided. The method is performed by a computing system. The method may be referred to as a computer-implemented method or as a data processing method. The method does not comprise a surgical step.

The method comprises obtaining medical image data of at least one anatomical element of a patient's body. The medical image data may be pre-operative medical image data. The medical image data may comprise or consist of X-ray image data, magnetic resonance (MR) image data or computer tomography (CT) image data.

The method further comprises obtaining planning data. The planning data may be part of a dataset comprising both the medical image data and the planning data.

The planning data is indicative of at least three planned positions associated with the at least one anatomical element. One or more (e.g., all) of the (e.g., at least three) planned positions are associated with at least one planned medical implant. The one or more (e.g., all) of the at least three planned positions may be associated with the at least one anatomical element by being associated with one or more medical implants to be implanted (e.g., according the planning data) into the at least one anatomical element and/or to be attached (e.g., according the planning data) to the at least one anatomical element and/or to be placed (e.g., according the planning data) in contact with the at least one anatomical element.

The method further comprises generating, based on the medical image data and the planning data, an image comprising a representation (e.g., a depiction or rendering) of the at least one anatomical element in a pose (e.g., at least one of a position and an orientation) defined by the at least three planned positions. The pose may be referred to as an alignment of the at least one anatomical element relative to a coordinate system of the generated image, for example relative to a vertical and/or horizontal image axis of the generated image. The pose may be determined without having to segment the at least one anatomical element. The pose of the at least one anatomical element may be defined by or determined based on only the planned positions associated with this anatomical element.

The image may be generated such that two or more of the at least three planned positions lie in a same (e.g., virtual or cross-sectional) plane corresponding to or being parallel to an image plane of the generated image.

Two or more of the planned positions may be associated with a same planned medical implant. At least two of the planned positions associated with the same planned medical implant may lie on a straight line defining an orientation of the same planned medical implant (e.g., in at least two or in three dimensions). The straight line may correspond to a longitudinal axis, a symmetry axis and/or an insertion trajectory of the same planned medical implant.

At least three of the planned positions associated with the same planned medical implant may lie in a plane defining an orientation of the same planned medical implant (e.g., in at least two or in three dimensions). The plane may correspond to an outer surface portion of the same planned medical implant, a center plane of the same planned medical implant, a symmetry plane of the same medical implant and/or a contact surface of the same planned medical implant. The plane comprising the at least three planned positions associated with the same planned medical implant may correspond to or be parallel to the image plane.

The image may be generated by orienting (e.g., rotating or aligning) or including (e.g., generating or projecting) the representation of the at least one anatomical element such that an angle between (i) a line (e.g., the straight line) indicating the orientation of the same planned medical implant and (ii) a vertical or horizontal image axis of the generated image fulfils at least one predefined criterion. The at least one predefined criterion may be that the angle is within a predefined range.

The image may be generated such that a plurality (e.g., all) of the planned positions associated with the same planned medical implant lie in a same plane corresponding to or being parallel to the image plane of the generated image. This same plane may comprise the plurality (e.g., all) of the planned positions associated with the same planned medical implant.

The at least three planned positions may comprise at least two pairs of planned positions, each pair being associated with a different planned medical implant.

The planned positions of each pair of planned positions may lie on a (e.g., respective) straight line defining an orientation of the planned medical implant associated with that pair. The straight line may correspond to a longitudinal axis, a symmetry axis and/or an insertion trajectory of the planned medical implant associated with that pair.

The image may be generated such that the planned positions of the at least two pairs (e.g., of all pairs) of planned positions lie in a same plane corresponding to or being parallel to the image plane of the generated image. The image plane may comprise the planned positions of the at least two pairs.

The image may be generated by orienting (e.g., rotating or aligning) or including the representation of the at least one anatomical element such that an angle between (i) an angle bisector that bisects an angle between (e.g., the straight) lines indicating orientations of the different medical implants and (ii) a vertical or horizontal image axis of the generated image fulfils at least one predefined condition. The at least one predefined condition may differ from or correspond to the at least one predefined criterion.

The medical image data may comprise volumetric image data. The image may be generated as a two-dimensional image representative of a cross-section (e.g., along the image plane) of the volumetric image data. The generated image may comprise a representation of (e.g., an outline of) at least a portion of (e.g., each of) the at least one planned medical implant (e.g., intersecting with the image plane).

The medical implant may comprise at least one component selected from (i) a spinal cage, (ii) a bone screw such as a pedicle screw, (iii) a pedicle fixation hook and (iv) a pedicle fixation band. The at least one anatomical element may comprise a bone such as a vertebra.

The method may further comprise storing the generated image. The method may further comprise triggering display of the generated image.

The method may further comprise obtaining user input indicative of a selected one of the at least one anatomical element. The user input may correspond to a tracked pose of a surgical instrument or pointer.

The image may be generated based on (e.g., only) the at least three planned positions associated with (e.g., only) the selected anatomical element.

For each of two or more of the at least one anatomical element, a respective image may be generated. Each of these images may be generated based on the medical image data and the planned positions associated with the respective anatomical element. A plurality of images (e.g., the respective images) may be generated based on the medical image data and the planning data (e.g., in, or by repeating, the step of generating the image), each image being generated based on at least three of the planned positions associated with a same respective anatomical element. Only the image generated based on the at least three of the planned positions associated with the selected anatomical element may be triggered to be displayed.

The method may comprise updating the image to be displayed depending on the user input. For instance, a first generated image including a representation of a first vertebra may be triggered to be displayed based on a first tracked pose of a pointer or surgical instrument as user input. Subsequently, a second generated image including a representation of a different, second vertebra may be triggered to be displayed based on a different, second pose of the pointer of surgical instrument as user input.

According to a second aspect, a system is provided. The system comprises at least one processor and may thus generally be referred to as a processing system. The at least one processor is configured to carry out the method according to the first aspect.

The system may further comprise at least one of the following components: a medical image acquisition unit configured to generate the medical image data; a user input device configured to acquire user input (e.g., defining one or more of the at least three planned positions and/or indicative of a subset of the planned positions and/or indicative of a selected one of the at least one anatomical element); a display unit configured to display at least the generated image.

According to a third aspect, a computer program is provided. The computer program comprises instructions which, when executed on at least one processor (e.g., the at least one processor of the system according to the second aspect), cause the at least one processor to carry out the method according to the first aspect. The computer program may be stored on or carried by a carrier medium. The carrier may be one of an electronic signal, an optical signal, a radio signal, or a (e.g., non-transitory) computer readable storage medium.

According to a fourth aspect, a storage medium is provided. The storage medium may be a non-transitory computer storage medium. The storage medium stores the computer program according to the third aspect.

### Brief Description of the Drawings

Further details, advantages and aspects of the present disclosure will become apparent from the following embodiments taken in conjunction with the drawings, wherein:
- Fig. 1: shows a schematic illustration of a system in accordance with the present disclosure;
- Fig. 2: shows a flowchart of a method in accordance with the present disclosure;
- Fig. 3: shows a rendering based on exemplary medical image data and indications of two planned medical implants;
- Fig. 4: shows the rendering of Fig. 3 and an indication of a plane;
- Fig. 5: shows a cross-sectional image along the plane indicated in Fig. 4 and indications of outlines of the two planned medical implants;
- Fig. 6: shows the cross-sectional image of Fig. 5 and an indication of an angle bisector;
- Fig. 7: shows the cross-sectional image of Fig. 6 rotated based on the angle bisector;
- Fig. 8: shows a spinal cage as a medical implant planned to be placed between two adjacent vertebrae; and
- Figs. 9a-9c: show a spinal sling and spinal clamps as planned medical implants to be attached to vertebrae.

### Detailed Description

In the following description, exemplary embodiments will be explained with reference to the drawings. The same reference numerals will be used to denote the same or similar structural features.

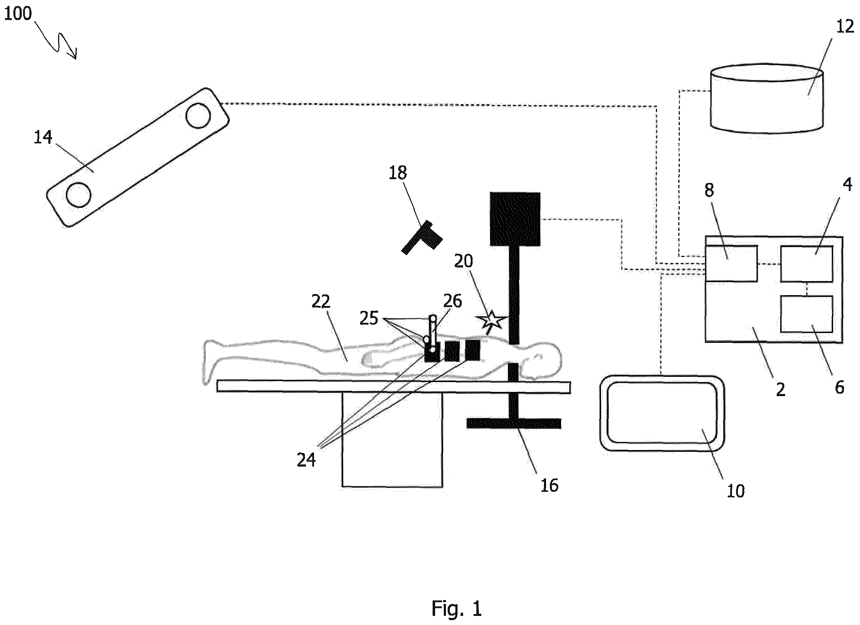

Fig. 1 shows a schematic illustration of a system 100 in accordance with the present disclosure. The system 100 comprises a computing unit 2 comprising at least one processor 4 communicatively connected to at least one memory 6 and at least one wired and/or wireless interface 8. The system 100 or the computing unit 2 may be generally referred to as "computing system". It is also possible that individual components of the computing unit 2 are part of a cloud computing platform, for example as virtual functions. The computing unit 2 may be part of a surgical navigation system.

The system 100 further comprises a display unit 10, a server or data storage 12, a tracking unit 14 and a medical image acquisition unit 16, each being coupled to the computing unit 2 via a wired or wireless connection. The display unit 10 may be a mounted on a stand in an operating room or be part of a head-mounted augmented reality display device. The data storage 12 may be part of a Picture Archiving and Communication System (PACS) of a hospital and may store medical image data such as X-ray images, CT image data and/or MR image data. The tracking unit 14 may comprise a (e.g., stereo-)camera or an electromagnetic field generator, and may be configured to track (e.g., localize) a user and/or an instrument 18 (e.g., comprising a tracking marker) located in a field of view of the tracking unit 14. The tracking unit 14 may be configured to track a patient marker 20 having a fixed spatial relationship to a patient's body 22. This may allow the computing unit 2 to determine a relative pose between the tracked instrument 18 and the patient's body 22. The medical image acquisition unit 16 may be a fluoroscopic imaging device, a C-arm scanner or a MR scanner, and may be configured to acquire or generate medical image data of at least a portion of the patient's body 22. The display 10 may be configured as a touchscreen to obtain user input. User input may also be obtained via the tracking unit 14, for example based on a pose of the tracked instrument 18. The patient's body 22 comprises a plurality of anatomical elements 24, for example bones such as vertebrae.

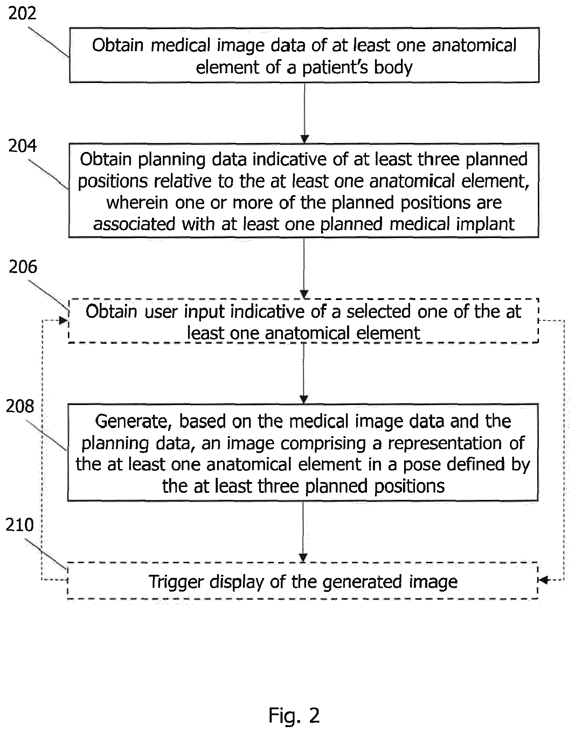

Fig. 2 shows a flowchart of a method in accordance with the present disclosure. The method may be performed by the system 100, for example by the at least one processor 4. The at least one memory 6 (e.g., configured as a non-transitory computer storage medium) may store a computer program comprising instructions which, when executed by the at least one processor 4, cause the at least one processor 4 to perform the method described herein.

The method illustrated in Fig. 2 is a method for generating an image including a representation of an anatomical element 24 of the patient's body 22.

The method comprises a step 202 of obtaining medical image data of (e.g., including) at least one anatomical element 24 of the patient's body 22.

The medical image data may be pre-operative medical image data. The medical image data may be referred to as (e.g., pre-operative) patient image data. The medical image data may comprise or consist of X-ray image data, magnetic resonance (MR) image data or computer tomography (CT) image data. The medical image data may comprise or consist of two-dimensional and/or three-dimensional image data. The medical image data may comprise or consist of volumetric image data.

The at least one anatomical element 24 may comprise a limb, a bone or an organ. Each of the at least one anatomical element 24 may have the same physical and/or biological properties. The physical properties may comprise one or more of: a transmittivity (e.g., for radiation used in generating the medical image data), an absorption (e.g., for radiation used in generating the medical image data), a water or hydrogen concentration. The biological properties may comprise one or more of: a type of biological material (e.g., bone, tissue or fluid), a type of tissue (e.g., nerval tissue or muscular tissue) or a biological function (e.g., a vascular function or a lymphatic function). In one variant, each of the at least one anatomical element 24 may comprise or be a bone, for example a vertebra.

The medical image data may be obtained from the medical image acquisition unit 16 (e.g., a C-arm scanner, a MR scanner or an X-ray apparatus). The medical image data may be obtained from the storage unit 12, which may be part of a PACS. Alternatively, the medical image data may be obtained from a portable storage device.

The method further comprises a step 204 of obtaining planning data.

The planning data may be obtained from the at least one memory 6, the data storage 12, the PACS system and/or from the portable storage unit. The planning data may be part of a dataset comprising both the medical image data and the planning data. The steps 202 and 204 of obtaining the medical image data and obtaining the planning data may thus be performed in unison. It is also possible to change the sequence of steps 202 and 204 compared with Fig. 2.

The planning data is indicative of at least three planned (e.g., spatial, two-dimensional or three-dimensional) positions 25 associated with the at least one anatomical element 24.

The at least three planned positions 25 may be associated with the at least one anatomical element 24 by lying on or inside the at least one anatomical element 24, by having a distance to the at least one anatomical element 24 that is lower than a predefined maximum distance and/or by being virtually linked to the at least one anatomical element 24, the virtual link (e.g., tag) being indicated by the medical image data and/or the planning data.

One or more (e.g., all) of the (e.g., at least three) planned positions 25 are associated with at least one planned medical implant 26.

Generally speaking, the one or more (e.g., all) of the at least three planned positions 25 may be associated with the at least one anatomical element 24 by being associated with one or more medical implants 26 to be implanted (e.g., according the planning data) into the at least one anatomical element 24 and/or to be attached (e.g., according the planning data) to the at least one anatomical element 24 and/or to be placed (e.g., according the planning data) in contact with the at least one anatomical element 24. A planned position 25 associated with a medical implant 26 may lie on the medical implant 26, for example on a distal or proximal end or tip of the medical implant 26. Alternatively, or in addition, a planned position 25 associated with a medical implant 26 may lie on an axis or plane defined by the medical implant 26 (e.g., a longitudinal axis, a symmetry axis, a planar contact surface or a symmetry plane).

The at least three planned positions 25 may be defined in an image coordinate system of the medical image data, or may be defined in a planning coordinate system having a known correlation with the image coordinate system. The known correlation may be referred to as a registration and/or may be defined by a transformation (e.g., comprising rotational and/or translational components) between the two coordinate systems. The at least three planned positions 25 may comprise a plurality of subsets of planned positions 25, each subset consisting of three or more planned positions 25 and being associated with a different one of the at least one anatomical element 24.

The method further comprises a step 208 of generating, based on the medical image data and the planning data, an image comprising a representation (e.g., a depiction or rendering) of the at least one anatomical element 24 in a pose (e.g., at least one of a position and an orientation) defined by the at least three planned positions 25.

The image may comprise the representation of exactly or only one of the at least one anatomical element 24. The pose may be referred to as an alignment of the at least one anatomical element 24 relative to a coordinate system of the generated image, for example relative to a vertical and/or horizontal image axis of the generated image. The pose may be determined without having to segment the at least one anatomical element 24. The pose of the at least one anatomical element may be defined exclusively or only by the at least three planned positions 25 associated with the at least one anatomical element represented or depicted in the generated image.

The image may be generated such that two or more of the at least three planned positions 25 lie in a same (e.g., virtual) plane corresponding to or being parallel to an image plane of the generated image. The image plane may correspond to a plane into which one or more objects (e.g., the at least one anatomical element 24 and/or the medical implant 26) are projected.

Two or more of the planned positions 25 may be associated with a same planned medical implant 26.

At least two of the planned positions 25 associated with the same planned medical implant 26 may lie on a straight line defining an orientation of the same planned medical implant 26 (e.g., in at least two or in three dimensions). The straight line may correspond to a longitudinal axis, a symmetry axis and/or an insertion trajectory of the same planned medical implant 26. The orientation of the same planned medical implant defined by the straight line may correspond to a longitudinal orientation, a main axis orientation, a symmetry axis orientation and/or an implantation direction.

At least three of the planned positions 25 associated with the same planned medical implant 26 may lie in a plane defining an orientation of the same planned medical implant 26 (e.g., in at least two or in three dimensions). The plane may correspond to or be parallel to an outer surface portion of the same planned medical implant 26, a center plane of the same planned medical implant 26, a symmetry plane of the same medical implant and/or a contact surface of the same planned medical implant 26. The orientation of the same planned medical implant 26 defined by the plane may correspond to an (e.g., angular or rotational) alignment or direction of the (e.g., plane of the) same medical implant 26.

The image may be generated by orienting (e.g., rotating or aligning) or including (e.g., generating or projecting) the representation of the at least one anatomical element 24 such that an angle between (i) a line (e.g., the straight line) indicating the orientation of the same planned medical implant 26 and (ii) a vertical or horizontal image axis of the generated image fulfils at least one predefined criterion. The at least one predefined criterion may be that the angle falls into a predefined range and/or is smaller than a predefined maximum angle (e.g., an angle of 10°, 8°, 5°, 2°, 1.5° or 1°). The at least one predefined criterion may be that the angle is minimized and/or zero. This may result in a generated image in which the planned medical implant has a desired orientation relative to the vertical and horizontal image axis, which may also depict the associated at least one anatomical element in a desired pose.

The image may be generated such that a plurality (e.g., all) of the planned positions 25 associated with the same planned medical implant 26 lie in a same plane corresponding to or being parallel to the image plane of the generated image. The image plane may comprise the plurality (e.g., all) of the planned positions associated with the same planned medical implant.

The at least three planned positions 25 may comprise at least two pairs of planned positions 25, each pair being associated with a different planned medical implant 26. The planned positions 25 of each pair of planned positions 26 may lie on a (e.g., respective) straight line defining an orientation of the planned medical implant 26 associated with that pair. The straight line may correspond to a longitudinal axis, a symmetry axis and/or an insertion trajectory of the planned medical implant 26 associated with that pair. The image may be generated such that the planned positions 25 of the at least two pairs (e.g., of all pairs) of planned positions 25 lie in the same image plane of the generated image. The image plane may comprise the planned positions 25 of the at least two pairs.

The image may be generated by orienting (e.g., rotating or aligning) or including the representation of the at least one anatomical element 24 such that an angle between (i) an angle bisector that bisects an angle between (e.g., the straight) lines indicating orientations of the different medical implants 26 and (ii) a vertical or horizontal image axis of the generated image fulfils at least one predefined condition. The at least one predefined condition may be that the angle is smaller than a predefined maximum angle (e.g., an angle of 10°, 8°, 5°, 2°, 1.5° or 1°). The at least one predefined condition may be that the angle is minimized and/or zero. The at least one predefined condition may differ from or correspond to the at least one predefined criterion. Instead of an angle bisector, an alignment line may be used which is determined such that distances between the alignment line and the planned positions, optionally weighted with respective priority values, are minimized. One may say that the alignment line may approximate the planned positions. Both approaches may result in an image in which multiple planned implants have desired poses relative to the vertical and horizontal image axis, which may also depict the associated at least one anatomical element in a desired pose.

The medical image data may comprise volumetric image data. The image may be generated as a two-dimensional image representative of a cross-section (e.g., along the image plane) of the volumetric image data. The image may be generated as a rendering representative of a (e.g., virtual) plane extending through the patient's body. The image may be a digitally rendered radiograph (DRR).

The generated image may comprise a representation of (e.g., an outline of) at least a portion of (e.g., each of) the at least one planned medical implant 26. The generated image may comprise a representation of a portion of the at least one planned medical implant 26 that intersects the image plane. The generated image may comprise an indication of an intersection area or an intersection outline of (e.g., each of) the at least one planned medical implant 26 with the image plane.

The medical implant 26 may comprise at least one component selected from (i) a spinal cage, (ii) a bone screw such as a pedicle screw, (iii) a pedicle fixation hook and (iv) a pedicle fixation band or pedicle fixation sling. The at least one anatomical element 24 may comprise a bone such as a vertebra.

The method may further comprise storing the generated image. The generated image may be stored in the at least one memory 6, the data storage unit 12 or another storage. The method may comprise transmitting the generated image to a receiver (e.g., the PACS system, a server or a user equipment such as a mobile phone).

The method may further comprise an optional step 210 of triggering display of the generated image. The image may be triggered to be displayed on the display unit 10 such as a screen, an augmented reality (AR) display, a head-mounted (e.g., augmented reality) display (HMD) or a tablet computer.

The method may further comprise an optional step 206 of obtaining user input indicative of a selected one of the at least one anatomical element. The user input may be obtained from a user interface (e.g., a touchscreen, a computer mouse, a joystick, a keyboard or a microphone). The user interface may be part of the display unit 10 or be realized with the tracking unit 14. That is, the user input may be obtained from the tracking unit 14. The user input may correspond to a tracked pose of the instrument or pointer 18. For example, the user may point to an anatomical element 24 using the pointer 18 to select this anatomical element 24. As another example, the user may orient the tracker instrument or pointer 18 in a pose associated with one of the planned medical implants (e.g., the pose aligning with an insertion trajectory of a planned pedicle screw) to select the anatomical element 24 associated with the one of the planned medical implants 26.

The image may be generated based on (e.g., only) the at least three planned positions 25 associated with (e.g., only) the selected anatomical element 24. That is, when generating the image, planned positions associated with other anatomical elements 24 may be disregarded.

For each of two or more of the at least one anatomical element 24, a respective image may be generated. Each of these images may be generated based on the medical image data and the planned positions 25 associated with the respective anatomical element 24. A plurality of images (e.g., the respective images) may be generated based on the medical image data and the planning data (e.g., in, or by repeating, the step of generating the image), wherein each image may be generated based on at least three of the planned positions 25 associated with a same respective anatomical element 24. Only the image generated based on the at least three of the planned positions 25 associated with the selected anatomical element 24 may be triggered to be displayed. That is, the selection of an anatomical element 24 may not only result in a corresponding image being triggered to be displayed, but may also result in a selection of one of a plurality of generated images triggered to be displayed.

The method may comprise updating the image (e.g., triggered to be) displayed depending on the user input (e.g., obtained in step 210). For instance, a first generated image including a representation of a first vertebra may be triggered to be displayed in step 210 based on a first tracked pose of a pointer or surgical instrument as user input obtained in step 206. Subsequently, a second generated image including a representation of a different, second vertebra may be triggered to be displayed in a subsequent iteration of step 210 based on a different, second pose of the pointer of surgical instrument obtained as user input at a later point in time (e.g., in a subsequent iteration of step 206).

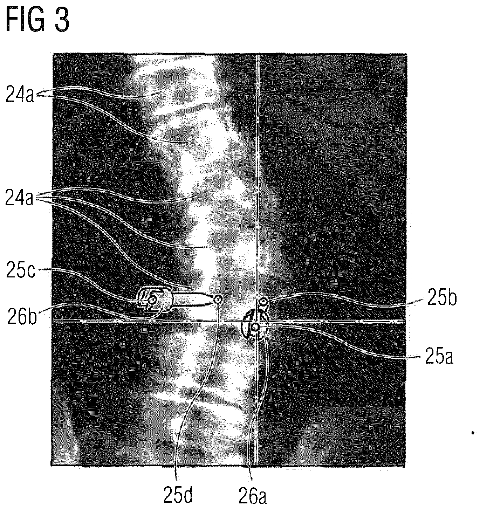

The method will now be explained based on specific examples with reference to Figs. 3 to 7.

Fig. 3 shows a rendering of medical image data of a portion of the patient's body 22, in this case a portion of the patient's spine. The medical image data in this example is pre-operative CT image data acquired from a CT scanner and the rendering represents a view in the posterior-anterior direction. One may say that the image plane of the rendering shown in Fig. 3 corresponds to the coronal or frontal plane. In the example of Fig. 3, the medical image data covers a plurality of vertebrae of the patient's body 22 as the anatomical elements 24.

Fig. 3 also illustrates pre-planned positions 25a-25d. The pre-planned positions 25a, 25b are associated with a first planned pedicle screw 26a as a first medical implant, and the preplanned positions 25c, 25d are associated with a second planned pedicle screw 26b as a second medical implant. Both implants 26a, 26b are planned to be implanted into a same vertebra 24. One may thus say that both implants 26a, 26b are associated with the same vertebra 24, and that the pre-planned positions 25a-25d are also associated with this same vertebra 24. In the example of Fig. 3, the planned position 25a corresponds to a head of the planned pedicle screw 26a and the planned position 25b corresponds to a tip of the same pedicle screw 26a. Thus, both positions 25a, 25b lie on a central longitudinal screw axis of the pedicle screw 26a. The same applies of the planned positions 25c, 25d lying on the central axis of the pedicle screw 26b. The positions 25a, 25b form a first pair and the positions 25c, 25d form a second pair.

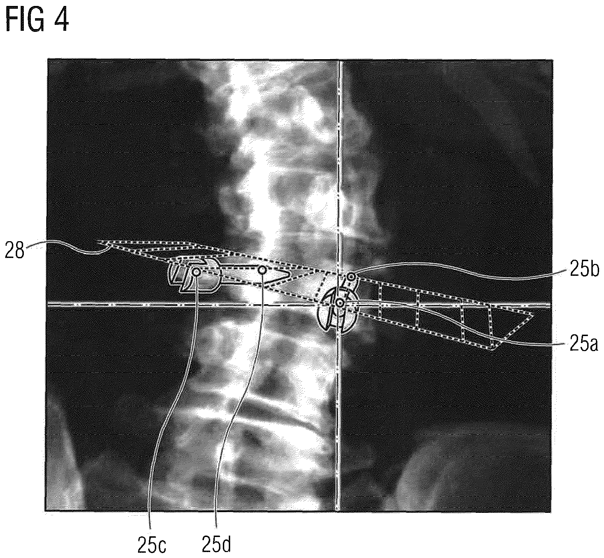

Fig. 4 illustrates the rendering of Fig. 3 and an indication of a plane 28. The plane 28 is defined by the pre-planned positions 25a-25d. In the illustrated example, all of the pre-planned positions 25a-25d lie within the plane 28.

In order to define the plane 28, at least three pre-planned positions 25 may be required. In case the two medical implants 26a, 26b do not lie within the same plane, two pre-planned positions (e.g., 25a, 25b) of one of the implants (e.g., 26a) may be used together with one of the preplanned positions (e.g., 25c) of the other one of the implants (e.g., 26b). Alternatively, two preplanned positions associated with a same medical implant 26 may be used in conjunction with one additional preplanned position 25 associated with the anatomical element 24 that is associated with this same medical implant 26. That is, all of the preplanned positions used for defining the plane 28 may be associated with the same anatomical element 24, and may or may not be associated with a medical implant 26. The one additional preplanned position 25 may correspond to a planned point that is located on an anatomical landmark, for example a point on the spinous process of the vertebra into which the same medical implant 26 is to be implanted, or to a point that is located on a planned trajectory. Other combinations of preplanned positions are also possible. The plane 28 may alternatively be defined such that none or only some of the preplanned positions 25 lie within the plane 28. For example, the plane 28 may be defined such that all preplanned positions have a similar distance thereto. The positions may be correlated priority values and distances to the plane 28 depending on these priority values. Other variants for determining the plane 28 are also possible.

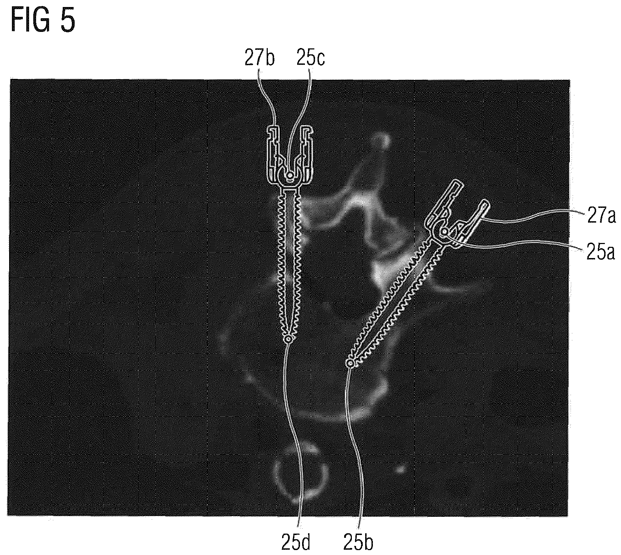

The plane 28 defines a cross-section through the volumetric CT image data of the patient's body 22 and corresponds to an image plane of the image to be generated. Fig. 5 shows a DRR generated based on the CT image data using the plane 28 as the image plane. In this example, both pedicle screws 26a, 26b and all pre-planned positions 25a-25d associated therewith lie within the image plane. Fig. 5 also indicates outlines 27a, 27b of the pedicle screws 26a, 26b that correspond to a line of intersection between the respective screw 26a, 26b and the plane 28. The generated image shown in Fig. 4 may be helpful for a user, as it not only represents the vertebra, bot also the implants 26a, 26b associated therewith. Nevertheless, the vertebra in this image may not have an orientation typically desired by a surgeon. It may be desired to generate or rotate the image such that the vertebra is oriented with the spinous process extending vertically.

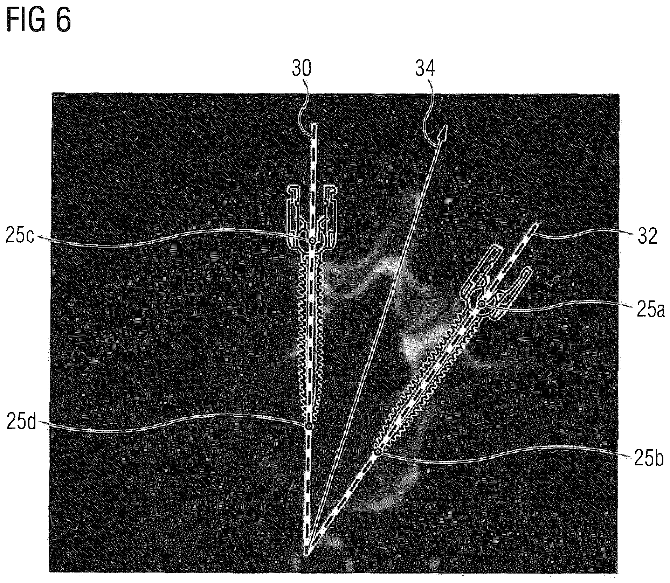

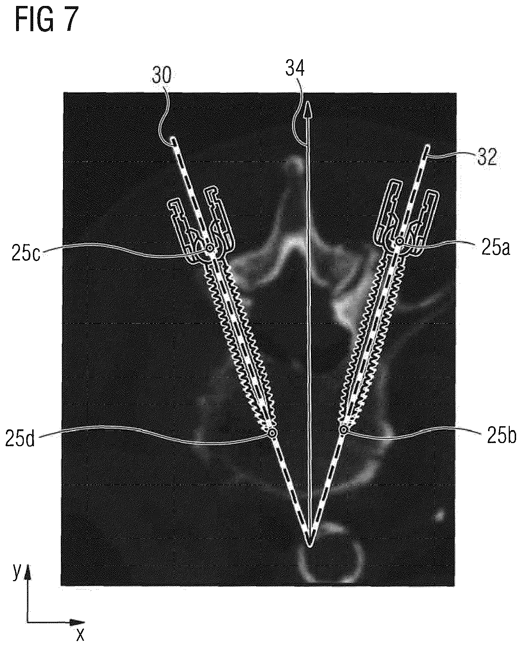

Instead of rotating the image of Fig. 5 manually, the present technique allows generating the image to be displayed such that the vertebra is represented in a pose defined by the pre-planned positions. As indicated in Fig. 6, straight lines 30, 32, each extending through one of the pairs of positions 25a, 25b and 25c, 25d may be used to this end. Namely, an angle bisector 34 between the two lines 30, 32 may be determined. This angle bisector is also indicated in Fig. 6. As shown in Fig. 7, the image may then be rotated or generated such that the angle bisector 34 aligns with the vertical image axis "y", i.e., an angle between the angle bisector 34 and the vertical image axis "y" is zero. This approach is particularly advantageous in case of spinal procedures involving planned pedicle screws, as such screws are typically aligned with similar angles relative to the spinous process due to the vertebra's symmetry.

The final image obtained with the method described herein may correspond to the image shown in Fig. 7, but the indication of the lines 30, 32 and the angle bisector 34 may be omitted therein. Instead of an angle bisector, a weighted average may be used in which more reliable planned positions are given more weight relative to less reliable planned positions. The reliability of the planned position (e.g., indicated as a priority value thereof) may be defined or determined based on the type of planned position (e.g., associated with a planned implant, associated with a landmark, associated with a planned trajectory, manually planned or automatically planned).

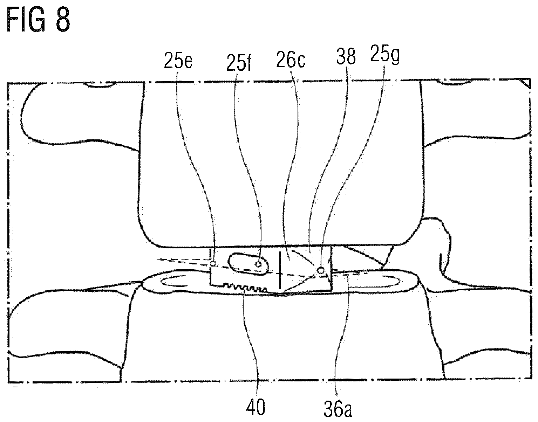

In the example of Fig. 8, the planned positions 25e-25g are associated with a spinal cage 26c. The planned positions 25e-25g lie in a middle symmetry plane 36a of the spinal cage 26c that is parallel to its outer contact surfaces 38, 40 configured to come into contact with adjacent vertebrae 24. The cage 26c has a given orientation within the middle symmetry plane, which orientation is defined by the longitudinal extension of the cage 26c and is also defined by the planned positions 25e-25g. In the illustrated example, the planned cage 26c points with one end, associated with position 25g, into a predefined and known anatomical direction. Thus, the orientation of the adjacent vertebrae 24 can be derived from the planned pose of the cage 26c, in particular based on the preplanned positions 25e-25g. This also allows generating the image such that the representation of the anatomical element (e.g., one of the vertebrae 24 adjacent to the cage 26c) has a desired pose (e.g., spinous process pointing upwards) in the image, the desired pose being defined by the preplanned positions 25e-25g and the known orientation of the plane 36a, namely, the predefined and known anatomical direction. This shows that the technique disclosed herein is not only applicable to pedicle screws, but also to spinal cages as medical implants 26.

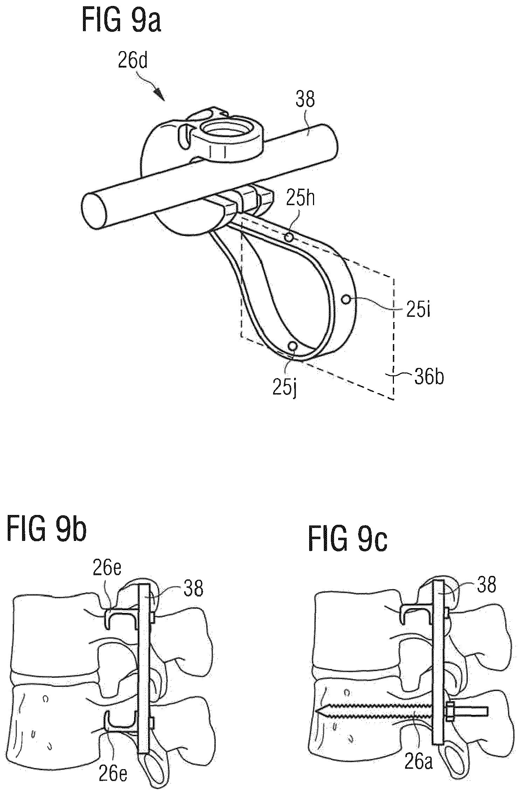

In Figs. 9a-9c, further examples of medical implants 26 are illustrated that could be used as medical implants having associated preplanned positions in the technique disclosed herein.

Fig. 9a shows a band or sling 26d configured to be attached to a vertebra. In this case, the preplanned positions 25h-25j may be located on the sling and may define a symmetry plane 36b of the sling. The sling may extend into a predefined and known anatomical direction, which means the symmetry plane 36b may have a predefined alignment relative to the vertebra to be attached to the sling. As in the case of the spinal cage 26c, this predefined alignment may be used together with the symmetry plane 36b to define the image plane and the desired rotation of the vertebra, such that the spinous process of the to-be-attached vertebra is oriented vertically in the generated image. Fig. 9a also shows a spinal rod 38 configured to be attached to the sling 26d.

Fig. 9b shows two pedicle hooks 26e, each associated with a different vertebra. The hooks 26e are joined via the spinal rod 38. Also in this case, each hook 26e may be associated with pre-planned positions (not shown) indicative of an orientation of the vertebra associated with the respective hook 26e. These positions may then be used to generate, for each of the vertebrae, a different image in which the respective vertebra is represented in the desired orientation with its spinous process pointing upwards.

Fig. 9c shows a combination of a pedicle hook 26e with a pedicle screw 26a, each associated with a different vertebra. The hook 26e is attached to the screw 26a via the rod 38. The technique described herein allows generating a respective image for each of the vertebrae depending on the medical implants associated with the respective vertebra, even if the implants are of different types as shown in Fig. 9c.

A user may move a tracked instrument 18 along the patient's spine. Based on the current position of the instrument 18, one of the vertebrae may be selected and the corresponding image may be displayed. This allows navigating the instrument 18 relative to the vertebrae based on images in which the respective vertebra always has the desired orientation, even if the real vertebrae of the patient are misaligned relative to one another due to scoliosis.

Generally speaking, medical conditions like e.g., scoliosis, deform the normal curvature of the spine, by twisting it and bring an abnormal curvature to the sides. The most common surgical operation for scoliosis is a spinal fusion. Spinal fusion involves realigning and fusing together the curved or misaligned vertebrae so that they heal into a single, solid bone. To do that, the surgeon may plan two pedicle screws 26a, 26b, pedicle slings 26d or pedicle hooks 26e for each vertebra that will serve as a passage for a rod 38 that will help restore the curvature of the spine during the healing process. Alternatively, or in addition, a spinal cage 26c to be inserted between vertebrae to be fused may be planned. At a later point, when using a navigation system to navigate a tracked instrument 18, the surgeon might struggle with an abnormal anatomical orientation, since the common screw planning and placement rules are defined and planned when looking at the vertebra in a desired, familiar orientation. Basically, the posterior side of the vertebra should be shown upwards in the image such that the spinous process extends vertically. This is how a healthy spine would look like with the patient laying on an operating bed in prone position. Aligning the views for each vertebra to this ideal orientation may allow for a smoother and more efficient planning and navigation process, since the user can quickly see the anatomy of interest in a familiar alignment.

The present disclosure may reduce complexity of this alignment step in the procedure through prediction of the preferred orientation based on pre-planned items (e.g., medical implants such as screws and k-wires, procedural information such as trajectories or user defined points of interest). Therefore, the user does not need to imagine nor take the time to manually re-orient the vertebra to its preferred anatomical orientation.

As has been explained above, a set of planning items (e.g., screws, k-wires, trajectories, points of interest) are planned, through automated processes and/or manual user definition. A plane is then constructed based on the orientation and position of these planning items or a subset thereof. A rotation orientation is constructed based on the orientation of these planning items or a subset thereof. A planning system orientation is defined which orients the world/patient to the constructed planning plane and rotation. This orientation allows the user to traverse patient data while keeping a preferred orientation of the view oriented to the plane and controlling the rotation of the view, for example such that a planning rotation is oriented upwards in the view. Multiple groups of these planning items can be constructed and grouped through automated processes and/or manual user definition. For example, planning items could be assigned to a vertebral level, even with no anatomical information about the vertebrae. For each of these groups, a planning system orientation can be constructed (e.g., a plane and a rotation). A user can orient the views to one or another of these planning system orientations, with switching between these orientations either through an automated process (e.g., based on a proximity of the tracked instrument 18 to the planning item) or manually.

As apparent from the above, the present disclosure provides for a technique that enables generating an image of at least one anatomical element of a patient's body in a preferred orientation. As the orientation is derived from planned positions indicated by planning data, no computationally expensive segmentation or image recognition algorithms may need to be employed to align the anatomical element in the preferred orientation. The image generated by the technique disclosed herein may also indicate the medical implants of interest for a user, in particular the medical implants planned for the at least one anatomical element depicted in the image. By selecting the generated image based on user input, for example based on a current tracked pose of a pointer or a surgical instrument relative to the patient's body, it may be ensured that the image to be displayed is indeed of interest for the user. Further modifications and advantages to the technique disclosed herein may become apparent to those skilled in the art.

## Claims

1. A method for generating an image including a representation of an anatomical element (24) of a patient's body (22), the method being performed by a computing system (2) and comprising:
obtaining (202) medical image data of at least one anatomical element (24) of a patient's body 822);
obtaining (204) planning data indicative of at least three planned positions (25) associated with the at least one anatomical element (24), wherein one or more of the planned positions (25) are associated with at least one planned medical implant (26);
generating (208), based on the medical image data and the planning data, an image comprising a representation of the at least one anatomical element (24) in a pose defined by the at least three planned positions (25).

2. The method of claim 1, wherein
the image is generated such that two or more of the at least three planned positions (25) lie in a same plane corresponding to or being parallel to an image plane of the generated image.

3. The method of claim 1 or 2, wherein
two or more of the planned positions (25) are associated with a same planned medical implant (26).

4. The method of claim 3, wherein
at least two of the planned positions (25a-25b; 25c-25d) associated with the same planned medical implant (26a; 26b) lie on a straight line (30; 32) defining an orientation of the same planned medical implant (26a; 26b).

5. The method of claim 3 or 4, wherein
at least three of the planned positions (25e-25g; 25f-25j) associated with the same planned medical implant (26c; 26d) lie in a plane (36a; 36b) defining an orientation of the same planned medical implant (26c; 26d).

6. The method of claim 4 or 5, wherein
the image is generated by orienting the representation of the at least one anatomical element (24) such that an angle between
(i) a line (30; 32) indicating the orientation of the same planned medical implant (26a; 26b) and
(ii) a vertical or horizontal image axis (x; y) of the generated image fulfils at least one predefined criterion.

7. The method of any one of claims 3 to 6, wherein
the image is generated such that a plurality of the planned positions (25) associated with the same planned medical (26) implant lie in a same plane (28; 36a; 36b) corresponding to or being parallel to the image plane of the generated image.

8. The method of any one of claims 1 to 7, wherein
the at least three planned positions (25a-25d) comprise at least two pairs of planned positions (25a-25b; 25c-25d), each pair being associated with a different planned medical implant (26a; 26b).

9. The method of claim 8, wherein
the planned positions of each pair of planned positions (25a-25b; 25c-25d) lie on a straight line (30; 32) defining an orientation of the planned medical implant (26a; 26b) associated with that pair.

10. The method of claim 8 or 9, wherein
the image is generated such that the planned positions (25a-25d) of the at least two pairs of planned positions (25a-25b; 25c-25d) lie in a same plane (28) corresponding to or being parallel to an image plane of the generated image.

11. The method of any one of claims 8 to 10, wherein
the image is generated by orienting the representation of the at least one anatomical element (24) such that an angle between
(i) an angle bisector (34) that bisects an angle between lines (30; 32) indicating orientations of the different medical implants (26a; 26b) and
(ii) a vertical or horizontal image axis (x; y) of the generated image fulfils at least one predefined condition.

12. The method of any one of claims 1 to 11, wherein
the medical image data comprises volumetric image data and the image is generated as a two-dimensional image representative of a cross-section of the volumetric image data.

13. The method of any one of claims 1 to 12, wherein the generated image comprises a representation of at least a portion of the at least one planned medical implant (26).

14. The method of any one of claims 1 to 13, wherein
the medical implant comprises at least one component selected from (i) a spinal cage (26c), (ii) a bone screw such as a pedicle screw (26a; 26b), (iii) a pedicle fixation hook (26e) and (iv) a pedicle fixation band (26d); and/or wherein
the at least one anatomical element comprises a bone such as a vertebra.

15. The method of any one of claims 1 to 14, further comprising:
triggering (210) display of the generated image.

16. The method of any one of claims 1 to 15, further comprising:
obtaining (206) user input indicative of a selected one of the at least one anatomical element (24),
wherein the image is generated based on the at least three planned positions (25) associated with the selected anatomical element (24).

17. The method of claims 15 and 16, wherein a plurality of images is generated based on the medical image data and the planning data, each image being generated based on at least three of the planned positions (25) associated with a same respective anatomical element (24), and only the image generated based on the at least three of the planned positions (25) associated with the selected anatomical element (24) is triggered to be displayed.

18. A system (2; 100) comprising at least one processor (4) configured to carry out the method according to any one of claims 1 to 17.

19. The system (100) according to claim 18, further comprising at least one of the following components:
a medical image acquisition unit (16) configured to generate the medical image data;
a user input device (10; 14) configured to acquire user input;
a display unit (10) configured to display at least the generated image.

20. A computer program comprising instructions which, when executed on at least one processor (4), cause the at least one processor (4) to carry out the method according to any one of claims 1 to 17, the computer program being optionally stored on a carrier medium (6; 12).

## Amended claims

### Amended claims in accordance with Rule 137(2) EPC.

1. A method for generating an image including a representation of an anatomical element (24) of a patient's body (22), the method being performed by a computing system (2) and comprising:
obtaining (202) medical image data of at least one anatomical element (24) of a patient's body 822);
obtaining (204) planning data indicative of at least three planned positions (25) associated with the at least one anatomical element (24), wherein one or more of the planned positions (25) are associated with at least one planned medical implant (26);
generating (208), based on the medical image data and the planning data, an image comprising a representation of the at least one anatomical element (24) in a pose defined by the at least three planned positions (25).

2. The method of claim 1, wherein
the image is generated such that two or more of the at least three planned positions (25) lie in a same plane corresponding to or being parallel to an image plane of the generated image.

3. The method of claim 1 or 2, wherein
two or more of the planned positions (25) are associated with a same planned medical implant (26).

4. The method of claim 3, wherein
at least two of the planned positions (25a-25b; 25c-25d) associated with the same planned medical implant (26a; 26b) lie on a straight line (30; 32) defining an orientation of the same planned medical implant (26a; 26b).

5. The method of claim 3 or 4, wherein
at least three of the planned positions (25e-25g; 25f-25j) associated with the same planned medical implant (26c; 26d) lie in a plane (36a; 36b) defining an orientation of the same planned medical implant (26c; 26d).

6. The method of claim 4 or 5, wherein
the image is generated by orienting the representation of the at least one anatomical element (24) such that an angle between
(i) a line (30; 32) indicating the orientation of the same planned medical implant (26a; 26b) and
(ii) a vertical or horizontal image axis (x; y) of the generated image fulfils at least one predefined criterion, wherein the predefined criterion is that the angle falls into a predefined range and/or is smaller than a predefined maximum angle.

7. The method of any one of claims 3 to 6, wherein
the image is generated such that a plurality of the planned positions (25) associated with the same planned medical (26) implant lie in a same plane (28; 36a; 36b) corresponding to or being parallel to the image plane of the generated image.

8. The method of any one of claims 1 to 7, wherein
the at least three planned positions (25a-25d) comprise at least two pairs of planned positions (25a-25b; 25c-25d), each pair being associated with a different planned medical implant (26a; 26b).

9. The method of claim 8, wherein
the planned positions of each pair of planned positions (25a-25b; 25c-25d) lie on a straight line (30; 32) defining an orientation of the planned medical implant (26a; 26b) associated with that pair.

10. The method of claim 8 or 9, wherein
the image is generated such that the planned positions (25a-25d) of the at least two pairs of planned positions (25a-25b; 25c-25d) lie in a same plane (28) corresponding to or being parallel to an image plane of the generated image.

11. The method of any one of claims 8 to 10, wherein
the image is generated by orienting the representation of the at least one anatomical element (24) such that an angle between
(i) an angle bisector (34) that bisects an angle between lines (30; 32) indicating orientations of the different medical implants (26a; 26b) and
(ii) a vertical or horizontal image axis (x; y) of the generated image fulfils at least one predefined condition, wherein the predefined condition is that the angle is smaller than a predefined maximum angle.

12. The method of any one of claims 1 to 11, wherein
the medical image data comprises volumetric image data and the image is generated as a two-dimensional image representative of a cross-section of the volumetric image data.

13. The method of any one of claims 1 to 12, wherein the generated image comprises a representation of at least a portion of the at least one planned medical implant (26).

14. The method of any one of claims 1 to 13, wherein
the medical implant comprises at least one component selected from (i) a spinal cage (26c), (ii) a bone screw such as a pedicle screw (26a; 26b), (iii) a pedicle fixation hook (26e) and (iv) a pedicle fixation band (26d); and/or wherein
the at least one anatomical element comprises a bone such as a vertebra.

15. The method of any one of claims 1 to 14, further comprising: triggering (210) display of the generated image.

16. The method of any one of claims 1 to 15, further comprising:
obtaining (206) user input indicative of a selected one of the at least one anatomical element (24),
wherein the image is generated based on the at least three planned positions (25) associated with the selected anatomical element (24).

17. The method of claims 15 and 16, wherein a plurality of images is generated based on the medical image data and the planning data, each image being generated based on at least three of the planned positions (25) associated with a same respective anatomical element (24), and only the image generated based on the at least three of the planned positions (25) associated with the selected anatomical element (24) is triggered to be displayed.

18. A system (2; 100) comprising at least one processor (4) configured to carry out the method according to any one of claims 1 to 17.

19. The system (100) according to claim 18, further comprising at least one of the following components:
a medical image acquisition unit (16) configured to generate the medical image data;
a user input device (10; 14) configured to acquire user input;
a display unit (10) configured to display at least the generated image.

20. A computer program comprising instructions which, when executed on at least one processor (4), cause the at least one processor (4) to carry out the method according to any one of claims 1 to 17, the computer program being optionally stored on a carrier medium (6; 12).
